# EUROPEAN PATENT SPECIFICATION

(11) **EP 2 193 767 B1**
(45) Date of publication and mention of the grant of the patent: **07.09.2011**
(21) Application number: 08105915.6
(22) Date of filing: 02.12.2008
(51) Int. Cl.: A61F 5/58

(54) **A device for treatment of stuttering**
Vorrichtung zur Behandlung von Stotterern
Dispositif pour le traitement du bégaiement

(43) Date of publication of application: 09.06.2010
(73) Proprietor: Oticon A/S, 2765 Smørum (DK)
(72) Inventor: Madsen, Anja Ravn, DK-2765, Smørum (DK)
(74) Representative: Nielsen, Hans Jörgen Vind

(56) References cited:
- WO-A-81/02513
- WO-A-94/00085
- WO-A-2004/028203
- WO-A-2005/037153
- WO-A-2006/060243
- DE-A1- 3 146 556
- DE-A1- 3 506 092
- US-A- 3 566 858
- US-A- 4 662 847
- US-A- 5 794 203
- US-A- 5 961 443
- US-A1- 2005 078 844
- US-A1- 2006 089 522

## Description

### TECHNICAL FIELD

The present invention relates to a device for providing aid against stuttering. The invention relates specifically to a portable listening system comprising a microphone device for picking up a person's voice and a listening device comprising a receiver.

The invention furthermore relates to use of a portable listening system.

The invention may e.g. be useful in applications involving listening devices to be worn by a person to be treated for stuttering.

### BACKGROUND ART

The prevalence of stuttering estimated to be between 0.7 and 1 % of the adult population.

Prior art devices that uses delayed sound for treatment of stuttering exist. There are, however, different problems with these, including:
- They are fairly expensive
- They are adapted for use only at home (e.g. with headphones, wire and external microphone), not suitable for direct face-to-face communication with others
- They don't offer a proper solution, when talking on the phone
- They do not provide a general solution for use by a hearing impaired.

It is known in the prior art to use a delay of own voice to influence stuttering. It is also known from WO 2005/037153 A1 to use a bone conducting hearing aid for that purpose.

Furthermore, a portable listening system according to the preamble of claim 1 is known from US 5,794,203.

### DISCLOSURE OF INVENTION

An object of the present invention is to provide a portable device for treatment of stuttering.

Objects of the invention are achieved by the invention described in the accompanying claims and as described in the following.

An object of the invention is achieved by a portable listening system according to claim 1.

According to the invention, the microphone device comprises an audio selection device for selecting an audio signal from a multitude of audio signals and forwarding the selected audio signal to the listening device.

The portable listening system is adapted to provide that a selected audio signal can be a signal from a telephone, e.g. a mobile telephone. Alternatively, the selected signal can be an audio signal from a PC, e.g. in connection with a telephone conversation conducted via the Internet (e.g. as an IP-telephone conversation or as part of a video conference or remote learning programme, such as a web-demo).

The portable listening system is adapted to provide that the delay of the user's own voice is automatically enabled when a telephone conversation is initiated.

An advantage of the invention is that it provides a flexible body worn system suitable for treatment of stuttering.

If a delay in the signal transmitted from an intermediate body worn communication device a comprising a microphone to a hearing aid is implemented, the combined system would be ideal to use for stutterers that seek treatment with delayed sound- especially stutterers with hearing loss can benefit from this system.

The combined system will give the wearer (hearing disabled or not) a system that can be used whenever wanted or needed - even when talking on the phone or in other situations, where no other solution is offered so far.

A hearing aid is nowadays so small that it is cosmetically to be preferred over e.g. headphones when in dialogue with others. Also, and more importantly, the speech from the wearer will be picked up and delayed by the intermediate communication device, whereas the speech from the person talking to you will be picked up - and not delayed - by the hearing aid. This will enable the stutterer to use the system in dialogue and in phone conversations with others.

In an embodiment, the microphone device and the listening device constitute two physically separate bodies, each having their own dedicated housing and either being not physically connected or physically connected by a releasable connecting mechanism, e.g. an electrical (and/or mechanical, e.g. acoustical) connector.

In an embodiment, the microphone device and the listening device are distinct, physically separate parts that only cooperate electromagnetically via a wireless (e.g. near-field, e.g. inductive, or far-field (radiated)) connection between them. No wired connections (or connectors) exist *between* the two devices.

In an embodiment, the listening device comprises a microphone for picking up an input sound.

In an embodiment, the portable listening system is adapted to provide that the voice of a person with whom the wearer of the portable listening system communicates is picked up by the microphone of the listening system. Thereby the wearer's own voice is delayed for the wearer, but the voice or sounds of the environment are not delayed.

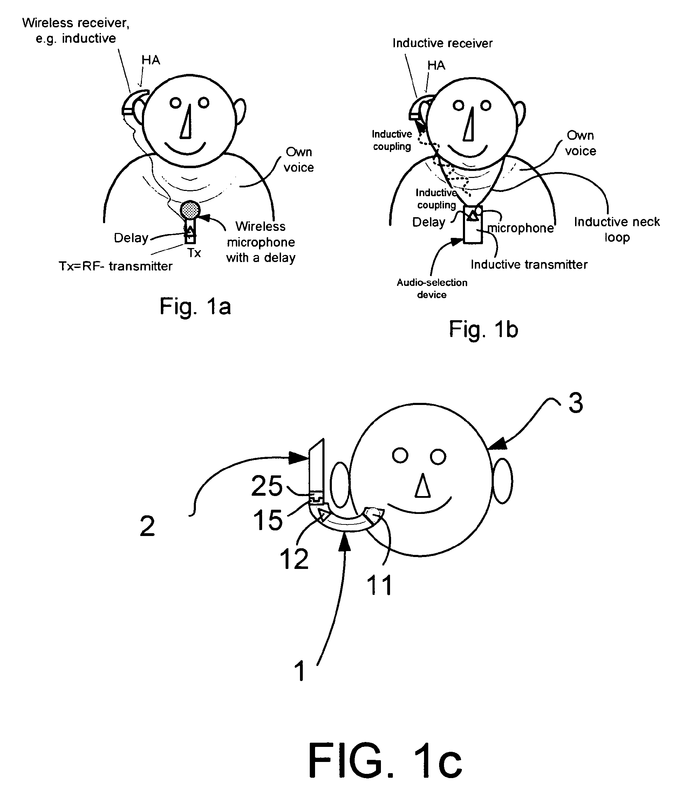

In an embodiment, the portable listening system is adapted to provide that the transmission between the microphone device and the listening device is wireless, e.g. based on inductive communication. In an alternative embodiment, the portable listening system is adapted to provide that the transmission between the microphone device and the listening device is wired, e.g. via an electrical connector, e.g. connecting a head mounted microphone with a listening device, e.g. a hearing instrument. Alternatively the microphone device may take the form of a click on-module adapted for being readily electrically connected to a listening device (e.g. like an FM-shoe, cf. e.g. FIG. 1c).

The signal path from input to output transducer of a hearing instrument has a certain minimum time delay. In general, the delay of the signal path is adapted to be as small as possible. In the present context, the term 'delay' or 'configurable/adjustable delay' is taken to mean an *additional* delay (i.e. in excess of the minimum delay of the signal path) that can be appropriately adapted to the user's needs.

In an embodiment, the portable listening system is adapted to provide that the (additional) delay is larger than 30 ms, such as in the range from 50 to 100 ms, such as larger than 100 ms, such as in the range from 100 ms to 500 ms, e.g. between 200 ms and 300 ms, such as larger than 500 ms.

In an embodiment, the delay is implemented as a buffer, either in hardware or software. In an embodiment an adjustable delay is implemented by having a number of buffers, each having a certain delay, and wherein the circuitry is adapted to be able to lead the (own voice) electrical signal through a configurable number of said buffers to create a certain delay in time of the (own voice) electrical signal (and thus of the acoustical signal presented to the user). In an embodiment, an adjustable delay is implemented as a software buffer, adjustable to a certain delay in steps between a minimum and a maximum value.

In an embodiment, the listening device is a hearing instrument. In an embodiment, the listening device is a hearing instrument for processing an input sound to an output sound according to a user's needs, the hearing instrument comprising an input transducer (e.g. a microphone) for converting an input sound to an electric input signal and an output transducer (e.g. a receiver) for converting a processed electric output signal to an output sound, a forward path being defined between the input transducer and the output transducer, the forward path comprising a signal processing unit adapted for providing a frequency-dependent gain and for providing a processed output signal.

In an embodiment, the portable listening system is adapted to provide that the delay is at least partially (such as that a major part of the delay is) implemented in the microphone device.

In an embodiment, the microphone device comprises a mobile telephone.

In an embodiment, the portable listening system is adapted to provide that an audio signal representing an incoming signal from a telephone, e.g. the voice of another person, is forwarded to the listening device without additional delay.

In an embodiment, the portable listening system is adapted to provide that a user can enable and/or disable the delay of the user's own voice.

In an embodiment, the portable listening system is adapted to provide that a user can enable a delay of an incoming signal from a telephone corresponding to and/or independent of the delay of the own voice signal.

In an embodiment, a microphone of the listening device is automatically disabled when a telephone conversation is initiated. Alternatively, the portable listening system comprises a manual switch for disabling a microphone of the listening device.

Use of a portable listening system as described above, in the section 'mode(s) for carrying out the invention', in the drawings and in the claims is furthermore provided by the present invention.

Further objects of the invention are achieved by the embodiments defined in the dependent claims and in the detailed description of the invention.

As used herein, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements maybe present, unless expressly stated otherwise. Furthermore, "connected" or "coupled" as used herein may include wirelessly connected or coupled. As used herein, the term "and/or" includes any and all combinations of one or more of the associated listed items. The steps of any method disclosed herein do not have to be performed in the exact order disclosed, unless expressly stated otherwise.

### BRIEF DESCRIPTION OF DRAWINGS

The invention will be explained more fully below in connection with a preferred embodiment and with reference to the drawings in which:
FIG. 1 shows various application setups of embodiments of a portable listening system according to the present invention;
FIG. 2 shows various partitions of a portable listening system according to the present invention; and
FIG. 3 shows an application scenario of an embodiment of a portable listening system according to the present invention.

The figures are schematic and simplified for clarity, and they just show details which are essential to the understanding of the invention, while other details are left out.

Further scope of applicability of the present invention will become apparent from the detailed description given hereinafter. However, it should be understood that the detailed description and specific examples, while indicating preferred embodiments of the invention, are given by way of illustration only, since various changes and modifications within scope of the invention will become apparent to those skilled in the art from this detailed description.

### MODE(S) FOR CARRYING OUT THE INVENTION

Fig. 1 shows various application setups of embodiments of a portable listening system according to the present invention.

FIG. 1a shows an embodiment of a portable listening system comprising a wireless microphone for picking up the voice of a person and converting the voice to an own voice electrical signal, a listening device, here in the form of hearing instrument (HA), the wireless microphone comprising a delay unit for delaying the own voice electrical signal and a transmitter for wirelessly transmitting a signal comprising the delayed own voice signal to a corresponding receiver in the hearing instrument. The wireless transmission can either be based on radiated fields between respective antennas of the wireless microphone and the hearing instrument or based on inductive coupling between respective induction coils. The wireless transmission can be analogue (e.g. FM) or digital (e.g. frequency hopping spread spectrum). The hearing instrument is adapted to receive the own voice electrical signal, to process it according to a user's hearing profile and to play it for the person by the receiver with a delay. The delay is adapted to be user programmable (e.g. by an adjustable activator, e.g. a turning knob or toggle switch or via a user interface on a display) e.g. in the range from 10 ms to 50 ms.

FIG. 1b shows an embodiment of a portable listening system comprising a communication device, here an audio selection device for selecting an audio signal from a multitude of audio signals and forwarding the selected audio signal to a listening device, here a hearing instrument (HA). The audio selection device comprises a microphone for picking up the voice of the person wearing the portable listening system and for converting the voice to an own voice electrical signal. The audio selection device comprises a delay unit for delaying the own voice electrical signal and a transmitter for wirelessly transmitting a signal comprising the delayed own voice signal to a corresponding receiver in the hearing instrument. In the present embodiment, the wireless transmission is based on inductive coupling between coils in the two devices or between a neck loop antenna distributing the field from a coil in the audio selecting device to the coil of the hearing instrument. Alternatively, the neck loop antenna can be driven directly from the transmitter of the audio selection device. The transmitted signal comprising the audio signal may be the baseband audio signal or a modulated carrier signal (e.g. a carrier modulated with the audio signal) or a digital signal comprising the audio signal.

The audio selection device is adapted to be able to mediate a telephone conversation, i.e. receive an incoming audio signal from a telephone (e.g. a mobile (or cellular) telephone) and to wirelessly forward it to the hearing instrument and to transmit the own voice signal picked up by the microphone of the audio selection device and transmit it to the telephone. The transmission to and from the telephone may e.g. be according to the Bluetooth protocol. In an embodiment, the received audio signal from a telephone is forwarded to the hearing instrument without additional delay, whereas the user's own voice picked up by the microphone of the audio selection device is delayed to counteract the user's stuttering. Alternatively, the received audio signal from a telephone is forwarded to the hearing instrument with the *same* additional delay as that applied to the own voice signal. In an embodiment, the microphone system of the hearing instrument is disabled during a telephone call, when the hearing instrument receives an audio signal from a telephone apparatus.

The wireless microphone of the embodiment of FIG. 1a and the microphone of the embodiment of FIG. 1b are specifically adapted to pick up the voice of the wearer of the portable listening system, e.g. in that it has a directionality that enables a direction towards the mouth of the wearer (when the system is appropriately mounted for normal operation).

FIG. 1c shows an embodiment of a portable listening system comprising a microphone device 1 comprising a microphone 11, here mounted on a longitudinal arm, like a headset microphone. The microphone device is adapted for being positioned close to a users' mouth when the microphone device is clicked on the listening device mounted at the ear of the user 3. The microphone device comprises a delay unit 12 electrically connected to the microphone 11. The delay unit is adapted for adjustably delaying the user's voice as picked up by the microphone. In another embodiment, the delay unit may be located in the listening device. In the embodiment of FIG.1c the connection between the microphone device 1 and the listening device 2 is not wireless, but the transmission from the microphone device 1 to the listening device 2 is conducted in the same way as from an FM shoe to a hearing aid via an electrical connector (comprising two matching electrical connectors15, 25, e.g. of a plug 25 of the listening device 2 and a socket 15 of the microphone device 1 (or vice versa)).

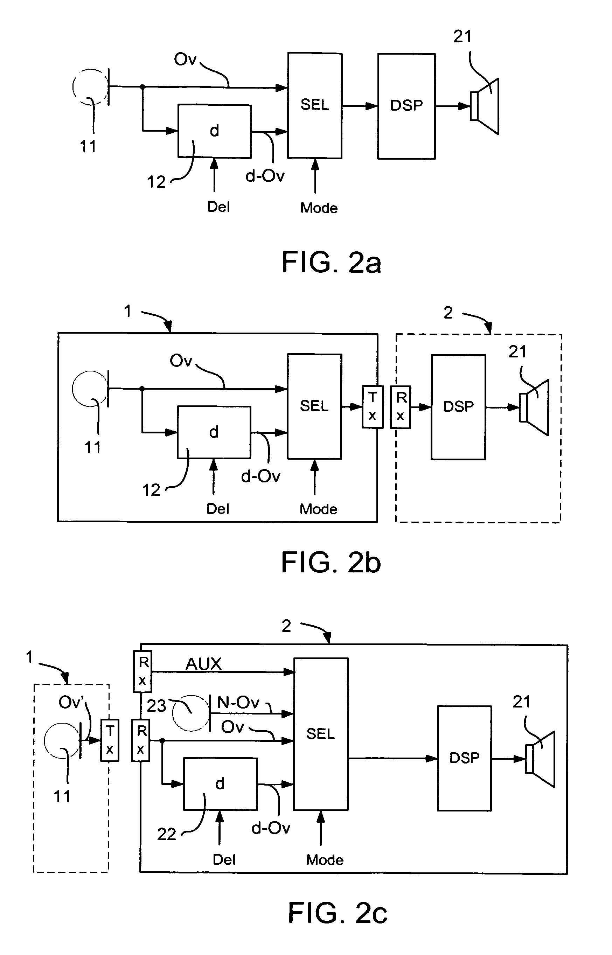

FIG. 2 shows various partitions of a portable listening system according to the present invention. The basic components of a portable listening system according to the present invention is a microphone device 1 for picking up a person's voice and a listening device for presenting sound to a user are shown in FIG. 2a. The system is adapted to pick up the voice of a person wearing the portable system by the microphone device, converting the voice to an own voice electrical signal, to transmit the own voice electrical signal to the listening device, to receive the own voice electrical signal in the listening device and to play it for the person by the receiver with a delay. In the embodiment of FIG. 2a, no partition between the microphone device (comprising at least the microphone 11) and the listening device (comprising at least the receiver 21) is indicated. The microphone 11 is adapted to converts a person's voice to an electrical own voice signal *Ov*, which is fed to a delay unit (*d*) 12 as well as to a selector unit (*SEL*). The delay unit 12 is adapted to delay the input signal *Ov* with an adjustable delay based on an input Del and to provide a delayed output signal *d-Ov,* which is fed to the selector unit (*SEL*). The selector unit is adapted to select one of its inputs *Ov* or *d-Ov* based on a mode input *Mode* and thus to provide a selected output signal (being either an undelayed or a delayed version of the own voice signal *Ov*), which (here) is fed to an optional processing unit (*DSP*) for further signal processing, e.g. for providing a frequency dependent gain, e.g. adapted to a user's needs. The processed output of the signal processing unit (*DSP*) is fed to the receiver 21 for converting the processed output signal to an output sound for being presented to the user of the portable listening system. The portable listening system may further comprise other functional blocks, such as an anti feedback system for minimizing the effect of acoustic feedback from the receiver to a microphone of the portable listening system.

FIG. 2b shows an embodiment of the portable listening system of FIG. 2a, wherein the system comprises 2 separate physical bodies, a microphone device 1 comprising microphone 11, delay unit 12 and selector unit (*SEL*) and a listening device comprising signal processor (DSP) and receiver 21. The two separate devices may be electrically connected, either by a wired connection or by a wireless connection. In an embodiment, a wireless link is setup by means of transceivers Tx and Rx in the microphone and listening devices, respectively. In FIG. 2b, the connection is indicated to be one-way from the microphone device 1 to the listening device 2 (transmission unit (Tx) in the microphone device 1 and reception unit (Rx) in the listening device 2). The link may alternatively be two-way (e.g. based on a plug and socket wired solution or a wireless connection, e.g. based on inductive communication or on radiated fields, e.g. analogue modulated link, e.g. an FM-link, or a digital link, according to a digital transmission standard, e.g. Bluetooth).

FIG. 2c shows another embodiment of the portable listening system of FIG. 2a, wherein the system is partitioned in 2 separate physical bodies. The microphone device 1 comprises microphone 11 adapted for picking up a users own voice and converting it to an own voice electrical signal *Ov*'. The microphone device further comprises a transceiver *Tx* adapted for (at least) transmitting the own voice electrical signal *Ov*' to the listening device. The listening device comprises delay unit 12, selector unit (*SEL*), signal processor (DSP) and receiver 21. Additionally the listening device (e.g. a hearing instrument) comprises a microphone 23 (or a microphone system comprising a number of microphones) for converting an acoustic sound to an electrical input signal *N*-*Ov*. Preferably, the microphone 23 is specifically adapted for providing a representation of sounds in the environment that do NOT originate from the user's own voice. The listening device further comprises a transceiver Rx (at least) for receiving an audio signal *AUX* from an auxiliary audio source (i.e. in addition to or alternatively to the microphone input(s)). The auxiliary audio input can e.g. be the incoming part of a telephone conversation, or any other incoming audio signal, e.g. from an entertainment device. In this embodiment the selector unit (*SEL*) is adapted to select as an output one of four inputs, 1) *AUX* from the auxiliary audio source, 2) *N-Ov* from the microphone 23 of the listening device, 3) *Ov* from the microphone device and representing the user's own voice and 4) *d-Ov* from the delay unit (*d*) 22 representing a delayed version of the user's own voice. The selector is adapted to select one of the inputs based on a mode select input (*Mode*). The signal AUX is selected when the user wishes to listen to an audio signal from an auxiliary audio source (e.g. a music player or an incoming telephone call). The signal N-Ov from the microphone 23 of the listening device is selected, when the microphone device 1 is not used (e.g. when the listening device, e.g. a hearing instrument, has its normal function, where the microphone 23 is the source of acoustic input to the listening device). The *Ov* signal from the microphone device 1 representing an undelayed version of the user's own voice is selected when the microphone device is used as a pick up of the user's own voice without introducing additional delay. The *d-Ov* signal from the delay unit (*d*) 22 representing a delayed version of the user's own voice is selected when the portable listening system is in its anti-stuttering learning mode. The transceiver Rx of the listening device for receiving an auxiliary input may be adapted to support a one- or a two-way link to/from the auxiliary device (e.g. a mobile telephone or an audio selection device). In case the microphone device 1 receives an incoming telephone call and forwards it to the listening device, the incoming part of the conversation and the delayed or undelayed version of the user's own vice picked up by the microphone device may be multiplexed (e.g. added) and transmitted over the same link to the listening device. In that case the listening device only needs one transceiver (optionally comprising only a *receiver,* if a fixed transmission channel is established).

In an embodiment, the selector unit in FIG. 2c is adapted to - at a given time - select one of the four inputs or to combine (e.g. add in an internal summation unit) two of more of the four inputs (e.g. AUX and *N-Ov* and (*Ov* OR *d-Ov*). This is preferable in an application where the microphone (system) 23 is adapted to pick up voices from the user's environment (preferably exclusive of the user's own voice) at the *same time* when an incoming telephone call is present at the AUX input AND the user's own voice is picked up by the microphone device (directly *Ov* or delayed *d-Ov*) *together* with that of the other voices (*N*-*Ov*) and where the combined signal is intended to be processed by the signal processor (DSP) and presented to the user via the receiver 21.

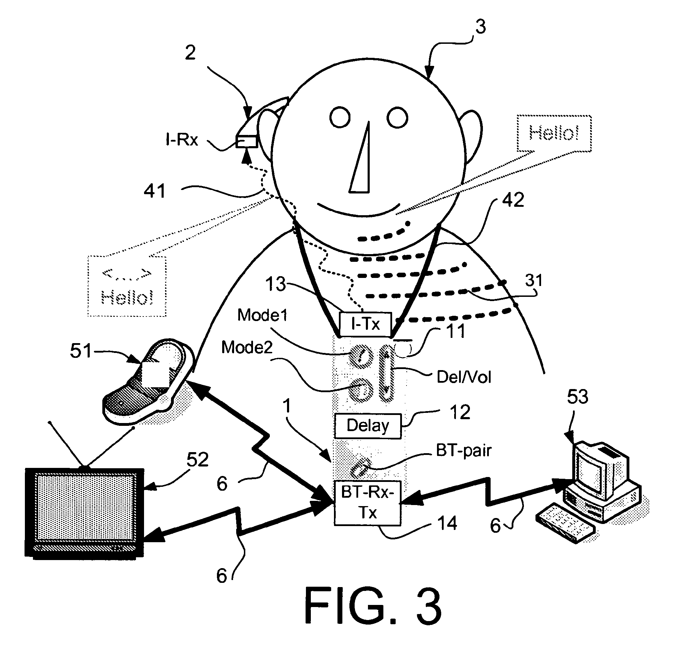

FIG. 3 shows an application scenario of an embodiment of a portable listening system according to the present invention, wherein the microphone device 1 comprises an audio selection device adapted for receiving a multitude of audio signals (here shown from an entertainment device, e.g. a TV 52, a telephone apparatus, e.g. a mobile telephone 51 and a computer, e.g. a PC 53). In the embodiment of FIG. 3 the microphone device comprising a microphone 11 adapted for picking up the user's own voice is connected to the external audio sources 51, 52, 53 via wireless links 6, here in the form of digital transmission links according to the Bluetooth standard as indicated by the Bluetooth transceiver 14 (*BT Tx-Rx*) in the microphone device 1. The audio sources and the microphone device may be paired using the button BT-pair. Once paired, the BT-address of the audio source may be stored in a memory of the microphone device for easy future pairing. The links may alternatively be implemented in any other convenient wireless and/or wired manner, and according to any appropriate transmission standard, possibly different for different audio sources. The microphone device 1 comprises a delay unit for applying a delay to the own voice electrical signal picked up by the microphone 11. The delay may be adjusted up and down in steps via a toggle button (*Del*/*Vol*), which at the same time may regulate volume of the audio signal transmitted to the listening device 2. The microphone device 1 further comprises a selector/combiner unit (not shown in FIG. 3) adapted for selecting and/or combining an appropriate signal or combination of signals for transmission to the listening device 2 as describe din connection with FIG. 2c above. The intended mode of operation can be selected by the user via mode selection buttons *Mode1* and *Mode2.* Here *Mode1* indicates a telephone conversation mode (where the audio signal from a currently actively paired mobile telephone is selected) and *Mode2* indicates an entertainment device mode (where the audio signal from a currently actively paired entertainment device is selected). The particular selected mode determines the signals to be selected/combined in the selector/combiner unit for transmission to the listening device. In Mode1, the own voice signal from microphone 11 AND the incoming signal from the mobile telephone is combined and transmitted to the listening device. In Mode 2, the audio signal from the selected entertainment device is selected and transmitted to the listening device. In a 'normal mode' where the neither a telephone call nor an auxiliary audio signal from an entertainment device is present, the own voice signal from the microphone 11 of the microphone device can be activated and transmitted to the listening device with or without additional delay at the wish of the user.

The listening device 2 is shown as a device mounted at the ear of a user 3. The listening device 2 of the embodiment of FIG. 3 comprises a wireless transceiver, here indicated to be based on inductive communication (*I-Rx*). The transceiver (at least) comprises an inductive receiver (i.e. an inductive coil, which is inductively coupled to a corresponding coil in a transceiver (I-Tx) of the microphone device 1), which is adapted to receive the audio signal from the microphone device (either as a baseband signal or as a modulated (analogue or digital) signal, and in the latter case to extract the audio signal from the modulated signal). The inductive link 41 between the microphone device and the listening device is indicated to be one-way, but may alternatively be two-way (e.g. to be able to exchange control signals between transmitting 1 and receiving 2 device, e.g. to agree on an appropriate transmission channel).

The microphone device 1 is shown to be carried around the neck of the user 3 in a neck-strap 42. The neck-strap 42 may have the combined function of a carrying strap and a loop antenna into which the audio signal from the microphone device is fed for better inductive coupling to the inductive transceiver of the listening device.

The anti-stuttering learning mode is illustrated in FIG. 3 by the acoustic signal ('Hello!') uttered by the user 3 in the form of acoustic waves 31 picked up by microphone 11 of the microphone device 1 and transmitted via the inductive link from the microphone device to the listening device (e.g. a hearing instrument or a headset) with a delay (`<....> Hello!'). The delay may be adjusted by the user (according to needs, e.g. based on the advice of an audiologist or on a trial and error basis), e.g. in steps of 10 ms from 0 to 300 ms.

An audio selection device, which may be modified and used according to the present invention is e.g. described in EP 1 460 769 A1 and in EP 1 981 253 A1.

The invention is defined by the features of the independent claim. Preferred embodiments are defined in the dependent claims. Any reference numerals in the claims are intended to be non-limiting for their scope.

Some preferred embodiments have been shown in the foregoing, but it should be stressed that the invention is not limited to these, but may be embodied in other ways within the subject-matter defined in the following claims.

### REFERENCES

- WO 2005/037153 A1 (ENTIFIC MEDICAL SYSTEMS) 28-04-2005
- EP 1 460 769 A1 (PHONAK) 22-09-2004
- EP 1 981 253 A1 (OTICON) 15-10-2008

## Claims

1. A portable listening system comprising a microphone device (1) for picking up a person's voice and a listening device (2) comprising a receiver (21), the system being adapted to pick up the voice of a person (3) wearing the portable system by the microphone device (1), converting the voice to an own voice electrical signal (Ov), to transmit the own voice electrical signal to the listening device (2), to receive the own voice electrical signal in the listening device (2), the system comprising a delay unit (d) allowing - in an anti-stuttering learning mode - to present the own voice electrical signal for the person (3) wearing the portable system by the receiver (21) with an adjustable delay, the microphone device (1) is comprising an audio selection device for selecting an audio signal from a multitude of audio signals and forwarding the selected audio signal to the listening device (2), the system being adapted to provide that a selected audio signal can be a signal from a telephone (51) **characterized in that** the delay of the user's own voice is automatically enabled when a telephone conversation is initiated.

2. A portable listening system according to claim 1 wherein the microphone device and the listening device constitute two physically separate bodies.

3. A portable listening system according to claim 1 or 2 wherein the listening device comprises a microphone for picking up an input sound.

4. A portable listening system according to claim 3 adapted to provide that the voice of a person with whom the wearer of the portable listening system communicates is picked up by the microphone of the listening device.

5. A portable listening system according to any one of claims 1-4 adapted to provide that the transmission between the microphone device and the listening device is wired, e.g. via corresponding electrical connectors, or wireless, e.g. based on inductive communication.

6. A portable listening system according to any one of claims 1-5 adapted to provide that the delay is larger than 30 ms, such as in the range from 50 to 100 ms, such as larger than 100 ms, such as in the range from 100 ms to 500 ms, e.g. between 200 ms and 300 ms, such as larger than 500 ms.

7. A portable listening system according to any one of claims 1-6 adapted to provide that the listening device is a hearing instrument.

8. A portable listening system according to any one of claims 1-7 adapted to provide that the delay is at least partially, such as that a major part of the delay is, implemented in the microphone device.

9. A portable listening system according to any one of claims 1-8 adapted to provide that the microphone device comprises an audio selection device for selecting an audio signal from a multitude of audio signals and forwarding the selected audio signal to the listening device in combination with the voice signal picked up by the microphone device.

10. A portable listening system according to any one of claims 1-9 adapted to provide that a selected audio signal can be a signal from a mobile telephone.

11. A portable listening system according to any one of claims 1-9 adapted to provide that the microphone device comprises a mobile telephone.

12. A portable listening system according to claim 10 or 11 adapted to provide that an audio signal representing an incoming signal from a telephone, e.g. the voice of another person, is forwarded to the listening device without additional delay.

13. A portable listening system according to any one of claims 1-12 adapted to provide that a user can enable and/or disable the delay of the user's own voice.

14. A portable listening system according to any one of claims 10-13 adapted to provide that the microphone of the listening device can be manually or automatically disabled when a telephone conversation is initiated.

## Patentansprüche

1. Tragbares Hörsystem mit einer Mikrofonvorrichtung (1) zum Aufnehmen einer Stimme einer Person und einer Hörvorrichtung (2) mit einem Hörer (21), wobei das System dazu ausgebildet ist, die Stimme einer das tragbare System tragenden Person (3) mit der Mikrofonvorrichtung (1) aufzunehmen, die Stimme in ein elektrisches Eigenstimmesignal (Ov) zu wandeln, das elektrische Eigenstimmesignal an die Hörvorrichtung (2) zu übertragen, das elektrische Eigenstimmesignal in der Horvorrichung (2) zu empfangen, wobei das System eine Verzögerungseinheit (d) aufweist, die es in einem Antistotterlernmodus erlaubt, das elektrische Eigenstimmesignal der das tragbare System tragenden Personen (3) mittels des Hörers (21) mit einer einstellbaren Verzögerung zu präsentieren, wobei die Mikrofonvorrichtung (1) eine Audioauswahlverrichtung zum Auswählen eines Audiosignals von einer Mehrzahl von Audiosignalen und zum Weiterleiten des ausgewählten Audiosignals an die Hörvorrichtung (2) aufweist, wobei das System ausgebildet ist, es vorzusehen, dass das ausgewählte Audiosignal ein Signal von einem Telefon (51) sein kann, wobei die Verzögerung der eigenen Stimme des Benutzers automatisch aktiviert wird, wenn ein Telefongespräch begonnen wird.

2. Tragbares Hörsystem gemäß Anspruch 1, wobei die Mikrofonvorrichtung und die Hörvorrichtung zwei physikalisch getrennte Körper bilden.

3. Hörvorrichtung gemäß Anspruch 1 oder 2, wobei die Hörvorrichtung ein Mikrofon zum Aufnehmen von Eingangslauten aufweist.

4. Tragbares Hörsystem gemäß Anspruch 3, das ausgebildet ist, es vorzusehen, dass die Stimme einer Person mit der der Träger des tragbaren Hörsystems kommuniziert mittels des Mikrofons der Hörvorrichtung aufgenommen wird.

5. Tragbares Hörsystem gemäß einem der Ansprüche 1 bis 4, das ausgebildet ist, es vorzusehen, dass die übertragung zwischen der Mikrofonvorrichtung und der Hörvorrichtung über Draht erfolgt, beispielsweise über entsprechende elektrische Anschlüsse, oder drahtlos, beispielsweise basierend auf induktiver Kommunikation.

6. Tragbares Hörgerät gemäß einem der Ansprüche 1 bis 5, das ausgebildet ist, es vorzusehen, dass die Verzögerung größer ist als 30 msec, beispielsweise im Bereich zwischen 50 bis 100 msec, beispielsweise größer als 100 msec, beispielsweise im Bereich zwischen 100 msec bis 500 msec, beispielsweise zwischen 200 msec und 300 msec, beispielsweise größer als 500 msec.

7. Tragbares Hörsystem gemäß einem der Anspruch 1 bis 6, das ausgebildet ist, es vorzusehen, dass die Hörvorrichtung ein Hörgerät ist.

8. Tragbares Hörsystem gemäß einem der Anspruch 1 bis 7, das ausgebildet ist, es vorzusehen, dass die Verzögerung zumindest teilweise, beispielsweise der Hauptteil der Verzögerung, in der Mikrofonvorrichtung implementiert ist.

9. Tragbares Hörsystem gemäß einem der Anspruch 1 bis 8, das ausgebildet ist, es vorzusehen, dass die Mikrofonvorrichtung eine Audioauswahlvorrichtung zum Auswählen eines Audiosignals aus einer Vielzahl von Audiosignalen und zum Weiterleiten des ausgewählten Audiosignals an die Hörvorrichtung in Kombination mit dem Aufnehmen des Stimmensignals durch die Mikrofonvorrichtung aufweist.

10. Tragbares Hörsystem gemäß einem der Anspruch 1 bis 9, das ausgebildet ist, es vorzusehen, dass ein ausgewähltes Audiosignal ein Signal von einem Mobiltelefon sein kann.

11. Tragbares Hörsystem gemäß einem der Anspruch 1 bis 9, das ausgebildet ist, es vorzusehen, dass die Mikrofonvorrichtung ein Mobiltelefon aufweist.

12. Tragbares Hörsystem gemäß Anspruch 10 oder 11, das ausgebildet ist, es vorzusehen, dass ein Audiosignal, das ein eingehendes Signal von einem Telefon, beispielsweise die Stimme einer anderen Person, repräsentiert, der Hörvorrichtung ohne zusätzliche Verzögerung zugeführt wird.

13. Tragbares Hörsystem gemäß einem der Ansprüche 1 bis 12, das ausgebildet ist, es vorzusehen, dass ein Nutzer die Verzögerung der Eigenstimme des Nutzers aktivieren oder deaktivieren kann.

14. Tragbares Hörsystem gemäß einem der Ansprüche 10 bis 13, das ausgebildet ist, es vorzusehen, dass Mikrofon der Hörvorrichtung manuell oder automatisch abgeschaltet werden kann, wenn ein Telefongespräch begonnen wird.

## Revendications

1. Système d'écoute portatif comprenant un dispositif de microphone (1) pour capter la voix d'une personne et un dispositif d'écoute (2) comprenant un récepteur (21), le système étant conçu pour capter la voix d'une personne (3) portant le système portatif à l'aide du dispositif de microphone (1), la voix étant convertie en un signal électrique de sa propre voix (Ov), pour transmettre le signal électrique de sa propre voix au dispositif d'écoute (2), dans le but de recevoir dans le dispositif d'écoute (2) le signal électrique de sa propre voix, le système comprenant une unité de temporisation (d) permettant - en mode apprentissage anti-bégaiement - au récepteur (21) de présenter avec un retard réglable le signal électrique de sa propre voix à la personne (3) portant le système portatif, le dispositif de microphone (1) comprenant un dispositif de sélection audio, destiné à sélectionner un signal audio parmi une multitude de signaux audio et à renvoyer le signal audio sélectionné au dispositif d'écoute (2), le système étant conçu pour faire en sorte qu'un signal audio sélectionné puisse être un signal provenant d'un téléphone (51), **caractérisé en ce que** le retard de la propre voix de l'utilisateur est automatiquement activé quand une conversation téléphonique est lancée.

2. Dispositif d'écoute portatif selon la revendication 1, dans lequel le dispositif de microphone et le dispositif d'écoute constituent deux éléments physiquement distincts.

3. Système d'écoute portatif selon la revendication 1 ou 2, dans lequel le dispositif d'écoute comprend un microphone pour capter un son d'entrée.

4. Système d'écoute portatif selon la revendication 3, conçu pour faire en sorte que la voix d'une personne avec laquelle communique le porteur du dispositif d'écoute portatif soit captée par le microphone du dispositif d'écoute.

5. Dispositif d'écoute portatif selon l'une quelconque des revendications 1 à 4, conçu pour faire en sorte que la transmission entre le dispositif de microphone et le Dispositif d'écoute soit câblée, par exemple par l'intermédiaire de connecteurs électriques correspondants, ou sans fil, par exemple en se fondant sur une communication inductive.

6. Dispositif d'écoute portatif selon l'une quelconque des revendications 1 à 5, conçu pour faire en sorte que le retard soit supérieur à 30 ms, notamment soit compris dans la plage de 50 à 100 ms, soit notamment supérieur à 100 ms, soit notamment compris dans la plage de 100 ms à 500 ms, par exemple entre 200 ms et 300 ms, et notamment soit supérieur à 500 ms.

7. Système d'écoute portatif selon l'une quelconque des revendications 1 à 6, conçu pour faire en sorte que le dispositif d'écoute soit un instrument auditif.

8. Système d'écoute portatif selon l'une quelconque des revendications 1 à 7, conçu pour faire en sorte que le retard soit au moins partiellement réalisé, et notamment qu'une partie principale du retard soit réalisée, dans le dispositif de microphone.

9. Dispositif d'écoute portatif selon l'une quelconque des revendications 1 à 8, conçu pour faire en sorte que le dispositif de microphone comprenne un dispositif de sélection audio destiné à sélectionner un signal audio parmi une multitude de signaux audio et à renvoyer le signal audio sélectionné au dispositif d'écoute en combinaison avec le signal vocal capté par le dispositif de microphone.

10. Système d'écoute portatif selon l'une quelconque des revendications 1 à 9, conçu pour l'aire en sorte qu'un signal audio sélectionné puisse être un signal provenant d'un téléphone mobile.

11. Système d'écoute portatif selon l'une quelconque des revendications 1 à 9, conçu pour faire en sorte que le dispositif de microphone comprenne un téléphone mobile.

12. Dispositif d'écoute portatif selon la revendication 10 ou 11, conçu pour faire en sorte qu'un signal audio représentant un signal d'entrée provenant d'un téléphone, par exemple la voix d'une autre personne, soit renvoyé au dispositif d'écoute sans retard additionnel.

13. Système d'écoute portatif selon l'une quelconque des revendications 1 à 12, conçu pour faire en sorte qu'un utilisateur puisse activer et/ou désactiver le retard de la propre voix de l'utilisateur.

14. Système d'écoute portatif selon l'une quelconque des revendications 10 à 13, conçu pour faire en sorte que le microphone du dispositif d'écoute puisse être manuellement ou automatiquement désactivé lors du lancement d'une conversation téléphonique.
